(19) Europäisches Patentamt
European Patent Office
Office européen des brevets

(11) Veröffentlichungsnummer: **0 221 847**
**A2**

# (12) EUROPÄISCHE PATENTANMELDUNG

(21) Anmeldenummer: 86810475.3

(22) Anmeldetag: 23.10.86

(51) Int. Cl.[4]: C 07 C 127/22
C 07 D 213/64, A 01 N 47/34

(30) Priorität: 29.10.85 CH 4640/85
17.09.86 CH 3722/86

(43) Veröffentlichungstag der Anmeldung:
13.05.87 Patentblatt 87/20

(84) Benannte Vertragsstaaten:
AT BE CH DE ES FR GB IT LI NL

(71) Anmelder: CIBA-GEIGY AG
Klybeckstrasse 141
CH-4002 Basel(CH)

(72) Erfinder: Drabek, Jozef, Dr.
Benkenstrasse 12
CH-4104 Oberwil(CH)

(72) Erfinder: Böger, Manfred
Wilhelm Glockstrasse 14
D-7858 Weil am Rhein 5(DE)

(54) **Salze von Benzoylharnstoffen.**

(57) Salze von Benzoylharnstoffen der Formel I

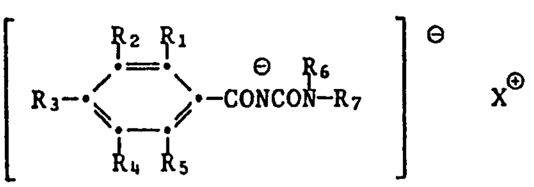

(I),

worin

$R_1$ bis $R_5$ unabhängig voneinander Wasserstoff, Halogen oder $C_1$-$C_6$-Alkoxy

$R_6$ Wasserstoff oder $C_1$-$C_6$-Alkyl

$R_7$ gegebenenfalls substituiertes Phenyl, Naphthyl, Pyridyl, Pyridazinyl, Pyrimidinyl oder Pyrazinyl und

$X^{\oplus}$ ein anorganisches oder organisches Kation bedeuten.

Verfahren zur Herstellung dieser Salze und ihre Verwendung in der Schädlingsbekämpfung werden beschrieben.

EP 0 221 847 A2

Croydon Printing Company Ltd.

0221847

CIBA-GEIGY AG
Basel (Schweiz)

5-15581/1+2

Salze von Benzoylharnstoffen

Die vorliegende Erfindung betrifft neuartige Salze von Benzoylharnstoffen, Verfahren zu ihrer Herstellung und ihre Verwendung in der Schädlingsbekämpfung.

Die Salze der Benzoylharnstoffe haben die Formel I

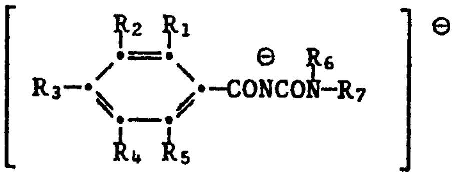 $X^{\oplus}$ (I),

worin

$R_1$ bis $R_5$ unabhängig voneinander Wasserstoff, Halogen oder $C_1$-$C_6$-Alkoxy,

$R_6$ Wasserstoff oder $C_1$-$C_6$-Alkyl,

$R_7$ gegebenenfalls substituiertes Phenyl, Naphthyl, Pyridyl, Pyridazinyl, Pyrimidinyl oder Pyrazinyl und

$X^{\oplus}$ ein anorganisches oder organisches Kation bedeuten.

Unter Halogen ist Fluor, Chlor, Brom oder Jod zu verstehen.

Die Alkyl- und Alkoxygruppen bei $R_1$-$R_6$ können geradkettig oder verzweigt sein und haben in der Kette vorzugsweise 1 bis 4 Kohlenstoffatome. Beispiele solcher Gruppen sind u.a.: Methyl, Aethyl, Propyl, Isopropyl, n-Butyl, n-Pentyl, n-Hexyl und deren Isomere.

Als anorganische Kationen kommen insbesondere die Kationen der Alkali- und Erdalkalimetalle, vorzugsweise das Natrium- und Kaliumkation, in Betracht.

Beispiele von organischen Kationen sind u.a.:

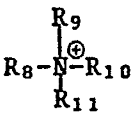 oder 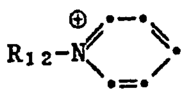 , worin $R_8$-$R_{11}$ unabhängig voneinander Wasserstoff, $C_1$-$C_{20}$-Alkyl, Benzyl oder Phenyl und $R_{12}$ Wasserstoff oder $C_1$-$C_{20}$-Alkyl bedeuten, wie z.B. folgende Kationen:

$(CH_3)_4N^{\oplus}$, $(C_2H_5)_4N^{\oplus}$, $(n\text{-}C_3H_7)_4N^{\oplus}$, $(i\text{-}C_3H_7)_4N^{\oplus}$, $(n\text{-}C_4H_9)_4N^{\oplus}$,

( 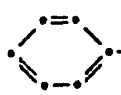 $-CH_2-)(CH_3)_3N^{\oplus}$, ( 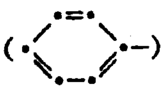 $-)(CH_3)_3N^{\oplus}$,

$CH_3-(CH_2)_n-$ 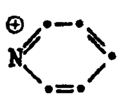 , $CH_3-(CH_2)_n-\overset{\oplus}{N}(CH_3)_3$,

wobei n eine Zahl 8 bis 15 bedeutet.

Folgende Substituenten an den Phenyl-, Naphthyl-, Pyridyl, Pyridazinyl-, Pyrimidinyl- oder Pyrazinylgruppen kommen bei $R_7$ u.a. in Frage: Halogenatome, insbesondere Fluor oder Chlor; Alkyl-, Halogenalkyl-, Alkoxy-, Halogenalkoxy-, Alkylthio-, Halogenalkylthio-, Alkenyl-, Halogenalkenyl-, Alkinyl-, Halogenalkinyl, Alkylsulfinyl-, Alkylsulfonyl-, Halogenalkylsulfinyl-, Halogenalkylsulfonyl-, Alkylamino- oder Dialkylaminogruppen; durch Halogen, Halogenalkyl, Halogenalkoxy oder Nitro substituierte Phenoxy- oder Pyridyloxygruppen;

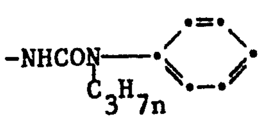 , $-O-CF_2-CF_2-O-$, $-O-CF_2-CFCl-O-$, Nitro oder Cyano.

Die Alkyl-, Alkoxy-, Alkenyl- und Alkinylreste in den oben angegebenen Substituenten haben bevorzugt 1 - 6 resp. 2 - 6 Kohlenstoffatome in der Kette, welche geradkettig oder verzweigt sein kann.

Bevorzugt sind Verbindungen der Formel I , worin

$R_1$ Fluor, Chlor oder Methoxy,

$R_2$ bis $R_5$ unabhängig voneinander Wasserstoff, Fluor, Chlor oder Methoxy,

$R_6$ Wasserstoff oder Methyl,

$R_7$ gegebenenfalls substituiertes Phenyl und

$X^{\oplus}$ ein Kation der Alkalimetalle,

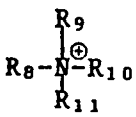 oder 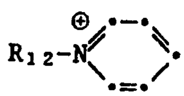 ,

$R_8$-$R_{11}$ unabhängig voneinander Wasserstoff, $C_1$-$C_{20}$-Alkyl, Benzyl oder Phenyl und

$R_{12}$ Wasserstoff oder $C_1$-$C_{20}$-Alkyl oder $X^{\oplus}$ insbesondere ein Kation der Alkalimetalle, $(CH_3)_4N^{\oplus}$, $(C_2H_5)_4N^{\oplus}$, $(n\text{-}C_3H_7)_4N^{\oplus}$, $(i\text{-}C_3H_7)_4N^{\oplus}$, $(n\text{-}C_4H_9)_4N^{\oplus}$,

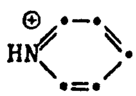 , 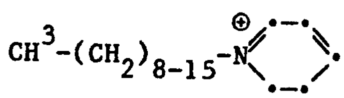 oder

$CH_3\text{-}(CH_2)_{8-15}\text{-}N^{\oplus}(CH_3)_3$ bedeuten.

Wegen ihrer biologischen Wirkung insbesondere bevorzugt sind Verbindungen der Formel I, worin

$R_1$ Fluor, Chlor oder Methoxy,

$R_2$ bis $R_5$ unabhängig voneinander Wasserstoff, Fluor, Chlor oder Methoxy,

$R_6$ Wasserstoff,

$R_7$ ein Phenyl substituiert durch ein oder mehrere Halogenatome, Halogenalkyl-, Halogenalkoxy- oder Dialkylaminogruppen oder durch einen durch ein oder mehrere Nitro, Halogen, Halogenalkyl oder Halogenalkoxy substituierten Phenoxy- oder Pyridyloxyrest oder durch die Gruppen

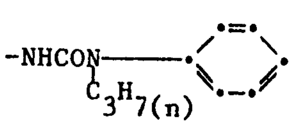 , $3,4\text{-}O\text{-}CF_2\text{-}CF_2\text{-}O\text{-}$ oder $3,4\text{-}O\text{-}CF_2\text{-}CFCl\text{-}O\text{-}$

und

$X^{\oplus}$ $Na^{\oplus}$, $K^{\oplus}$, oder $(CH_3)_4N^{\oplus}$, $(C_2H_5)_4N^{\oplus}$, $CH_3-(CH_2)_{15}-N^{\oplus}(CH_3)_3$ oder $(n-C_4H_9)_4N^{\oplus}$ bedeuten.

Beispiele von erfindungsgemässen Benzoylharnstoff-Salzen der Formel I sind u.a.:

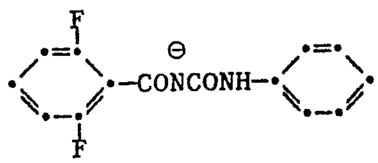

$Na^{\oplus}$

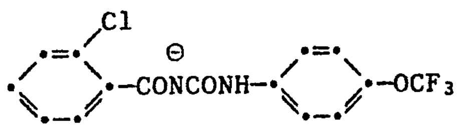

$K^{\oplus}$

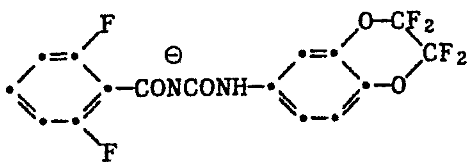

$(n-C_4H_9)_4N^{\oplus}$

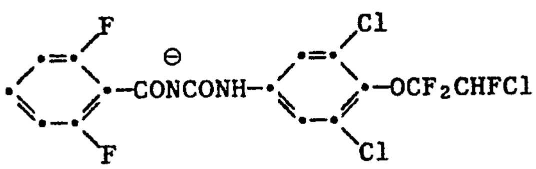

$(C_2H_5)_4N^{\oplus}$

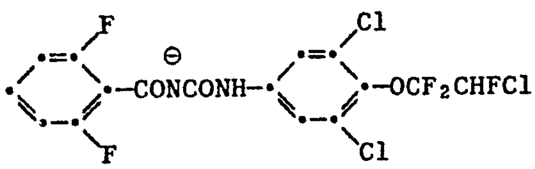

$CH_3-(CH_2)_{15}-N^{\oplus}(CH_3)_3$

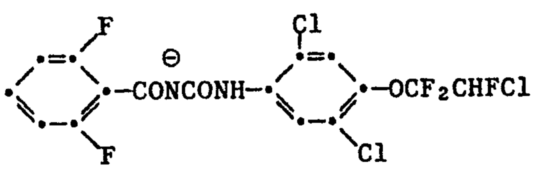

$(n-C_4H_9)_4N^{\oplus}$

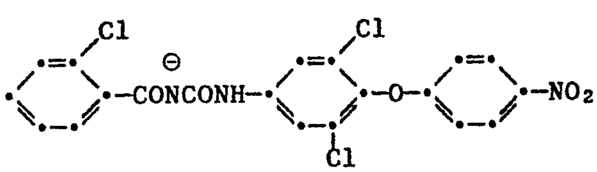

$Na^{\oplus}$

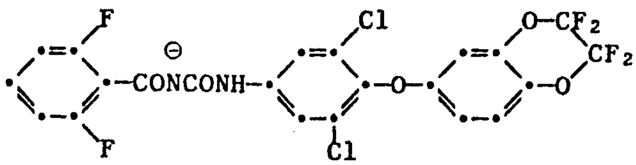

$(n-C_4H_9)_4N^{\oplus}$

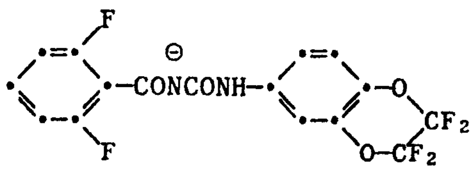

$(CH_3)_4N^{\oplus}$

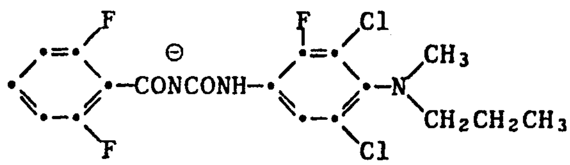

$(n-C_4H_9)_4N^{\oplus}$

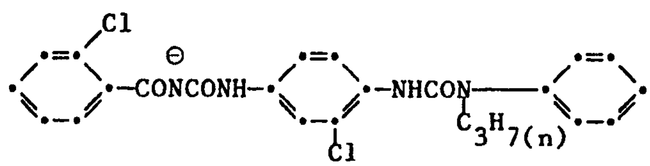

$(CH_3)_4N^{\oplus}$

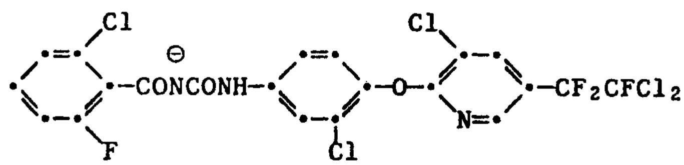

$K^{\oplus}$

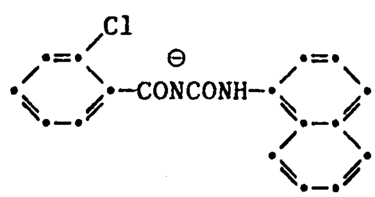

$(n-C_4H_9)_4N^{\oplus}$

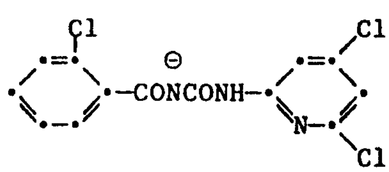

$(n-C_4H_9)_4N^{\oplus}$

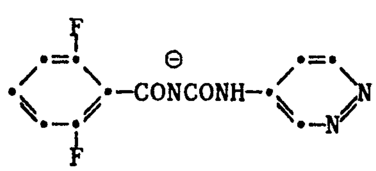

$(CH_3)_4N^{\oplus}$

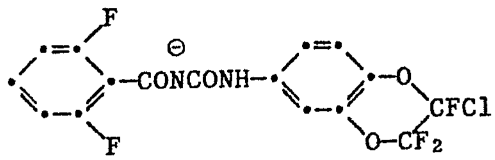
Na$^{\oplus}$

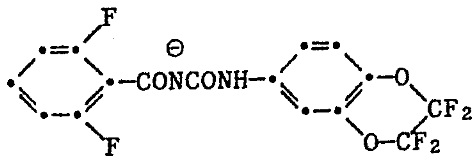
Na$^{\oplus}$

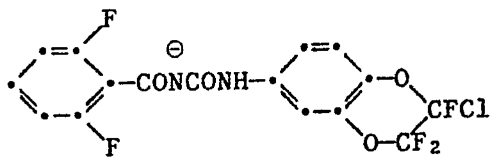
K$^{\oplus}$

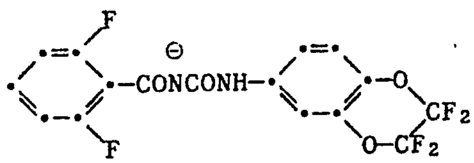
K$^{\oplus}$

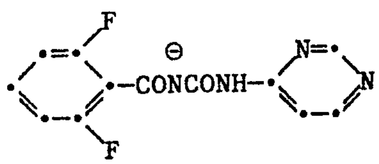
Na$^{\oplus}$

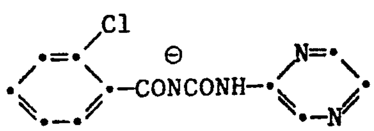
K$^{\oplus}$

Die erfindungsgemässen Salze der Formel I werden in an sich bekannter Weise hergestellt, z.B. durch Umsetzen einer Verbindung der Formel II

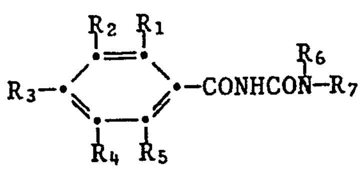
(II),

worin $R_1$-$R_7$ die für die Formel I angegebene Bedeutung haben, mit einem Metallalkanolat, wie z.B. Natriumäthylat oder Kaliummethylat oder mit entsprechenden Ammoniumhydroxiden der Formel III

$X^{\oplus}OH^{\ominus}$ (III),

worin $X^{\oplus}$ ein organisches Kation bedeutet.

Die Salzbildung wird vorzugsweise bei Raumtemperatur und in Gegenwart eines Alkohols, vorzugsweise Methanol oder Aethanol, durchgeführt.

Die Harnstoffe der Formel II sind bekannt oder können nach bekannten Methoden hergestellt werden.

Ueberraschenderweise wurde gefunden, dass die vorliegenden Verbindungen der Formel I bei guter Pflanzenverträglichkeit und geringer Warmblütertoxizität ausgezeichnete Wirksamkeit als Schädlingsbekämpfungsmittel aufweisen. Sie eignen sich vor allem zur Bekämpfung von Pflanzen und Tiere befallenden Insekten und Vertretern der Ordnung Akarina.

Insbesondere eignen sich die Verbindungen der Formel I zur Bekämpfung von Insekten der Ordnungen: Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera, sowie von Vertretern der Ordnung Akarina der Familien: Ixodidae, Argasidae, Tetranychidae und Dermanyssidae.

Neben ihrer Wirkung gegenüber Fliegen, wie z.B. Musca domestica, und Mückenlarven können Verbindungen der Formel I auch zur Bekämpfung von pflanzenschädigenden Frassinsekten in Zier- und Nutzpflanzungen, insbesondere in Baumwollkulturen (z.B. Spodoptera littoralis und Heliothis virescens), sowie in Obst- und Gemüsekulturen (z.B. Laspeyresia pomonella, Leptinotarsa decemlineata und Epilachna varivestis) eingesetzt werden. Die Verbindungen der Formel I zeichnen sich durch eine ausgeprägte larvizide und ovo-larvizide Wirkung gegen Insekten, insbesondere Larven von fressenden Schadinsekten, aus. Werden Verbindungen der Formel I von adulten Insekten-Stadien

mit dem Futter aufgenommen, so ist in vielen Fällen, insbesondere bei Coleopteren, wie z.B. Anthonomus grandis, eine verminderte Ei-Ablage und/oder reduzierte Schlupfrate festzustellen.

Verbindungen der Formel I haben auch eine günstige Wirkung gegen Bodeninsekten (z.B. Aulacophora femoralis, Chortophila brassicae, Diabrotica balteata, Pachnoda savignyi und Scotia ypsilon).

Die Verbindungen der Formel I können ferner zur Bekämpfung von Ektoparasiten, wie Zecken und Lucilia sericata, an Haus- und Nutztieren eingesetzt werden, z.B. durch Tier-, Stall- und Weidebehandlung.

Die Verbindungen der Formel I eignen sich ferner zur Bekämpfung der folgenden Obst- und Gemüsekulturen befallenden Milbenspezies: Tetranychus urticae, Tetranychus cinnabarinus, Panonychus ulmi, Bryobia rubrioculus, Panonychus citri, Eriophyes piri, Eriophyes ribis, Eriophyes vitis, Tarsonemus pallidus, Phyllocoptes vitis und Phyllocoptruta oleivora.

Die Verbindungen der Formel I können vorteilhaft als Beizmittel zur Behandlung von Saatgut und Vorrat (Früchte, Knollen, Körner) und Pflanzenstecklingen und zur Saatfurchenapplikation zum Schutz vor Pilzinfektionen und Insekten sowie gegen die im Erdboden auftretenden phytopathogenen Pilze und Insekten eingesetzt werden.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und können daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Salze der Formel I zeichnen sich nicht nur durch hohe akarizide und insektizide Wirkung, sondern auch durch gute Löslichkeit in Lösungs- und Verdünnungsmitteln sowie durch verbesserte Formulierbarkeit aus.

Die Formulierung, d.h. die den Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie

N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäure-methyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8 - 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Ricinusölthioxilat, Poly-propylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1979;
Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel I oder Kombinationen dieser Wirkstoffe mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Herstellungsbeispiel 1

a) Herstellung des Natriumsalzes vom N-(2,6-Difluorbenzoyl)-N'-[3,5-dichlor-4-(1',1',2',2'-tetrafluoräthoxy)-phenyl]-harnstoff.
9,2 g N-(2,6-Difluorbenzoyl)-N'-[3,5-dichlor-4-(1'-1',2',2'-tetrafluoräthoxy)-phenyl]-harnstoff werden in 30 ml Methanol suspendiert. Unter Rühren wird zu dieser Suspension eine Lösung von 0,45 g Na in 20 ml Methanol zugetropft. Die Lösung wird noch 30 Minuten bei 20°C gerührt. Nach dem Abdestillieren des Methanols erhält man die Verbindung Nr. 1 der Formel

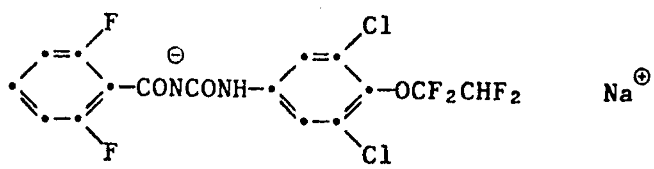

mit einem Schmelzpunkt von 153°C.

b) Herstellung des Tetrabutylammoniumsalzes vom N-(2,6-Difluorbenzoyl)-N'[3,5-dichlor-4-(3'-chlor-5'-trifluormethylpyridyloxy-(2)-phenyl]-harnstoff.
5,4 g N-(2,6-Difluorbenzoyl)-N'-[3,5-dichlor-4-(3'-chlor-5'-trifluor-methylpyridyloxy-(2)-phenyl]-harnstoff werden in 60 ml Methanol suspendiert. In dieser Suspension wird unter Rühren bei 20°C eine Lösung von 2,6 g Tetrabutylammoniumhydroxid in Methanol (10,4 g einer 25%igen Lösung) getropft. Die entstandene klare Lösung wird 30 Minuten bei 20°C gerührt. Nach dem Abdestillieren des Methanols wird der Rückstand mit Hexan verrührt, das kristalline Produkt abgenutscht und im Vakuum bei 30°C getrocknet. Man erhält die Verbindung Nr. 2 der Formel

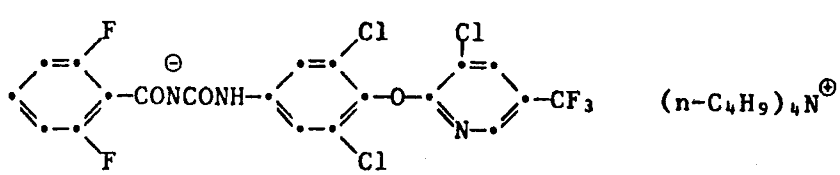

mit einem Schmelzpunkt von 104 - 108°C.

Auf analoge Weise werden auch die folgenden Verbindungen der Formel I hergestellt:

| Verb. Nr. | | | Smp. [°C] |
|---|---|---|---|
| 3 | 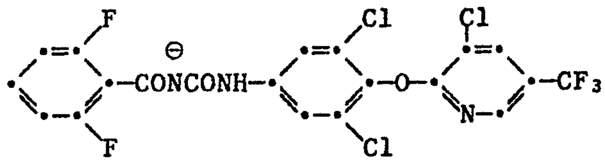 | $Na^{\oplus}$ | 158° |
| 4 | 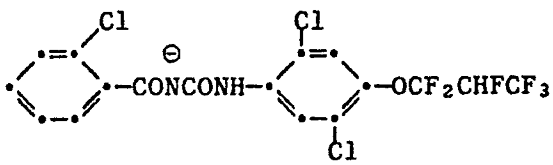 | $Na^{\oplus}$ | 140° |
| 5 | 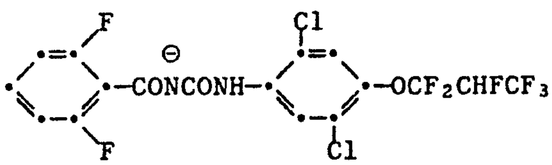 | $Na^{\oplus}$ | 169-171° |
| 6 | 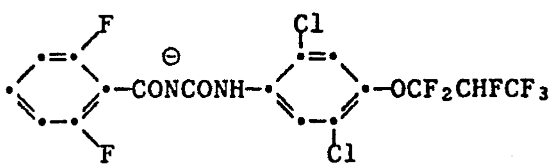 | $K^{\oplus}$ | 195-200° |
| 7 | 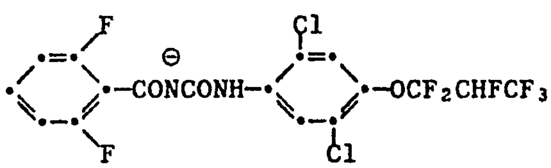 | $(n\text{-}C_4H_9)_4N^{\oplus}$ | 105-110° |
| 8 | 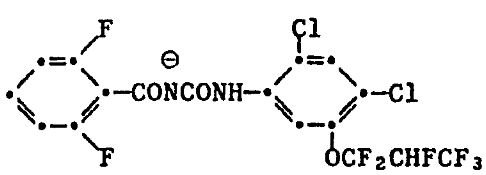 | $(n\text{-}C_4H_9)_4N^{\oplus}$ | 108-112° |

| Verb. Nr. | | | Smp. [°C] |
|---|---|---|---|
| 9 | 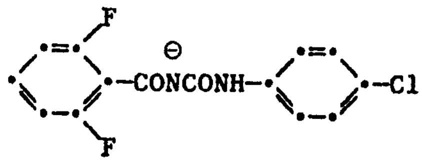 | $Na^{\oplus}$ | > 260° |
| 10 | 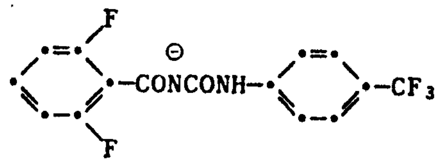 | $K^{\oplus}$ | > 260° |
| 11 | 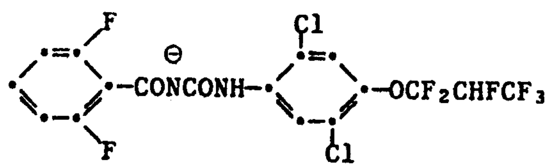 | $(C_2H_5)_4N^{\oplus}$ | glasartige Masse |
| 12 | 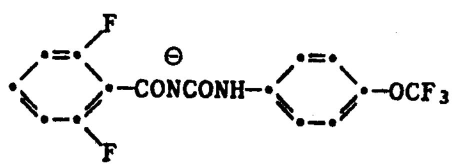 | $K^{\oplus}$ | 170° |
| 13 | 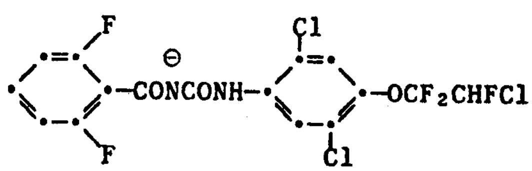 | $(n\text{-}C_4H_9)_4N^{\oplus}$ | 144-146° |

| Verb. Nr. | | | Smp. [°C] |
|---|---|---|---|
| 14 | 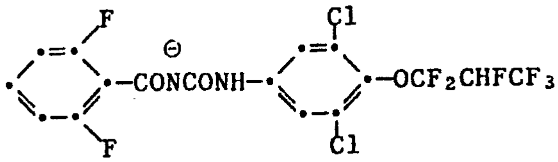 | $Na^{\oplus}$ | 145° |
| 15 | 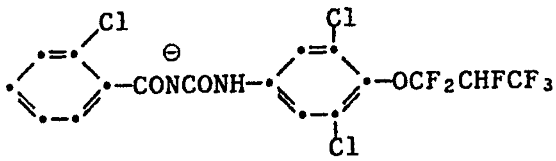 | $(C_2H_5)_4N^{\oplus}$ | gelbes Oel |
| 16 | 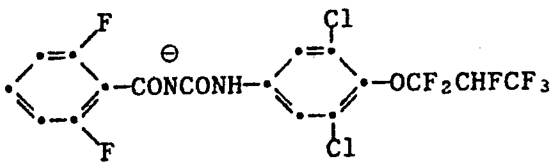 | $(n\text{-}C_4H_9)_4N^{\oplus}$ | gelbes Oel |
| 17 | 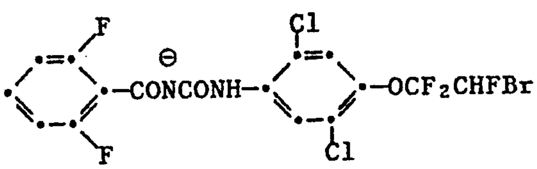 | $(n\text{-}C_4H_9)_4N^{\oplus}$ | 135° |
| 18 | 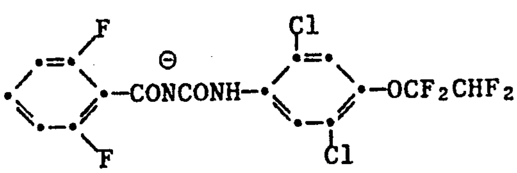 | $Na^{\oplus}$ | 174-175° |
| 19 | 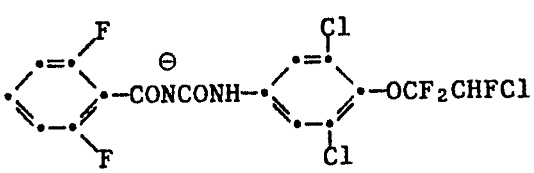 | $(n\text{-}C_4H_9)_4N^{\oplus}$ | 146-148° |

| Verb. Nr. | | | Smp. [°C] |
|---|---|---|---|
| 20 | 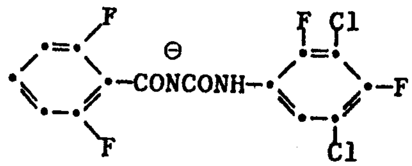 | $Na^{\oplus}$ | 232-240° |
| 21 | 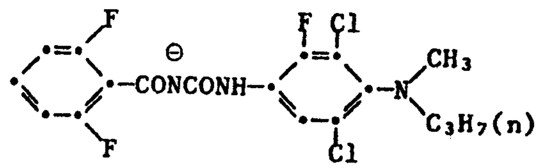 | $Na^{\oplus}$ | 160-162° |
| 22 | 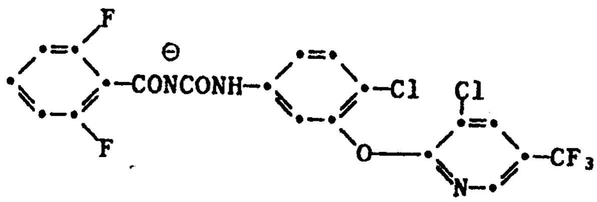 | $Na^{\oplus}$ | 185° |
| 23 | 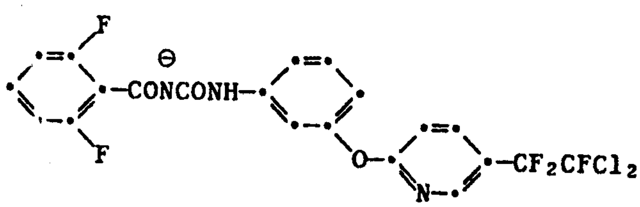 | $Na^{\oplus}$ | 100° |
| 24 | 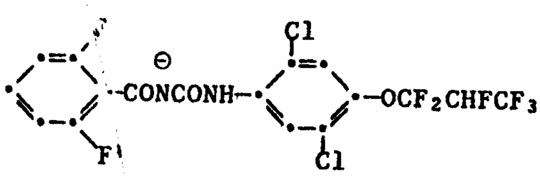 | $(n\text{-}C_4H_9)_4N^{\oplus}$ | 105-110° |

Beispiel 2: Formulierungsbeispiele für Wirkstoffe gemäss dem Herstellungsbeispiel 1 (% = Gewichtsprozent)

| 2.1 Emulsions-Konzentrate | a) | b) | c) | d) |
|---|---|---|---|---|
| Wirkstoff gemäss dem Herstellungsbeispiel 1 | 10 % | 25 % | 40 % | 50 % |
| Ca-Dodecylbenzolsulfonat | - | 5 % | 8 % | 6 % |
| Ricinusöl-polyäthylenglykoläther (36 Mol AeO) | - | 5 % | - | - |
| Tributylphenol-polyäthylenglykoläther (30 Mol AeO) | - | - | 12 % | 4 % |
| Ricinusölthioxilat | 25 % | - | - | - |
| Cyclohexanon | - | - | 15 % | 20 % |
| Butanol | 15 % | - | - | - |
| Xylolgemisch | - | 65 % | 25 % | 20 % |
| Essigester | 50 % | - | - | - |

Aus solchen Konzentraten können durch Verdünnen mit Wasser Emulsionen jeder gewünschten Konzentration hergestellt werden.

| 2.2 Lösungen | a) | b) |
|---|---|---|
| Wirkstoff gemäss dem Herstellungsbeispiel 1 | 10 % | 5 % |
| Aethylenglykol-monomethyläther | - | - |
| Polyäthylenglykol (MG 400) | 70 % | - |
| N-Methyl-2-pyrrolidon | 20 % | - |
| Epoxidiertes Kokosnussöl | - | 1 % |
| Benzin (Siedegrenzen 160 - 190°C) | - | 94 % |

Die Lösungen sind zur Anwendung in Form kleinster Tropfen geeignet.

| 2.3 Granulate | a) | b) |
|---|---|---|
| Wirkstoff gemäss dem Herstellungsbeispiel 1 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.4 Stäubemittel | a) | b) | c) | d) |
|---|---|---|---|---|
| Wirkstoff gemäss dem Herstellungsbeispiel 1 | 2 % | 5 % | 5 % | 8 % |
| Hochdisperse Kieselsäure | 1 % | 5 % | - | - |
| Talkum | 97 % | - | 95 % | - |
| Kaolin | - | 90 % | - | 92 % |

Durch inniges Vermischen der Trägerstoffe mit dem Wirkstoff erhält man gebrauchsfertige Stäubemittel.

| 2.5 Spritzpulver | a) | b) | c) |
|---|---|---|---|
| Wirkstoff gemäss dem Herstellungsbeispiel 1 | 20 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7 - 8 Mol AeO) | - | 2 % | - |
| Hochdisperse Kieselsäure | 5 % | 10 % | 10 % |
| Kaolin | 67 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

2.6 Extruder Granulat

| | |
|---|---|
| Wirkstoff gemäss dem Herstellungsbeispiel 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

2.7 Umhüllungs-Granulat

| | |
|---|---|
| Wirkstoff gemäss dem Herstellungsbeispiel 1 | 3 % |
| Polyäthylenglykol (MG 200) | 3 % |
| Kaolin | 94 % |

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

2.8 Suspensions-Konzentrat

| | |
|---|---|
| Wirkstoff gemäss dem Herstellungsbeispiel 1 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 3: Biologiebeispiele für die Verbindungen gemäss dem Herstellungsbeilspiel 1

Beispiel 3.1: Wirkung gegen Musca domestica:

Je 50 g frisch zubereitetes Nährsubstrat für Maden wird in Becher eingewogen. Von einer 1 gew.-%igen acetonischen Lösung des betreffenden Wirkstoffes wird eine bestimmte Menge auf das in den Bechern befindliche Nährsubstrat pipettiert, so dass sich eine Wirkstoffkonzentration von 800 ppm ergibt. Nach dem Durchmischen des Substrates lässt man das Aceton mindestens 20 Stunden lang verdampfen.

Dann werden pro Wirkstoff je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert. Die pro Ansatz ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen der Formel I gemäss Beispiel 1 zeigen gute Wirkung in obigem Test.

Beispiel 3.2: Wirkung gegen Lucilia sericata:

Zu 9 ml eines Zuchtmediums wird bei 50°C 1 ml einer 0,5 Gew.-% Aktivsubstanz enthaltenden wässrigen Zubereitung hinzugefügt. Nun werden ca. 30 frisch geschlüpfte Lucilia sericata-Larven zum Zuchtmedium gegeben, und nach 48 und 96 Stunden wird die insektizide Wirkung durch Ermittlung der Abtötungsrate festgestellt.

Verbindungen der Formel I gemäss Beispiel 1 zeigen in diesem Test gute Wirkung gegen Lucilia sericata.

Beispiel 3.3: Wirkung gegen Aëdes aegypti:
Auf die Oberfläche von 150 ml Wasser, das sich in einem Behälter befindet, wird so viel einer 0,1 gew.-%igen acetonischen Lösung des Wirkstoffes pipettiert, dass eine Konzentration von 800 ppm erhalten wird. Nach Verdunsten des Acetons wird der Behälter mit 30 bis 40 2-tägigen Aëdes-Larven beschickt. Nach 1, 2 und 5 Tagen wird die Mortalität geprüft.

Verbindungen der Formel I gemäss Beispiel 1 zeigen in diesem Test gute Wirkung gegen Aëdes aegypti.

Beispiel 3.4: Insektizide Frassgift-Wirkung:
Ca. 25 cm hohe eingetopfte Baumwollpflanzen werden mit wässrigen Wirkstoffemulsionen besprüht, die den Wirkstoff jeweils in ansteigenden Konzentrationen bis zu 100 ppm enthalten.

Nach dem Antrocknen des Sprühbelages werden die Baumwollpflanzen mit Spodoptera littoralis- bzw. Heliothis virescens-Larven im dritten larvalen Stadium besiedelt. Der Versuch wird bei 24°C und 60 % relativer Luftfeuchtigkeit durchgeführt. Nach 120 Stunden wird die Mortalität der Test-Insekten in Prozenten bestimmt.

Eine Wirkung von 80 - 100 % (Mortalität) zeigen die Verbindungen Nr. 1, 2, 3, 22 und 23 bei 12,5 ppm gegen Spodoptera- und Heliothis-Larven.

Beispiel 3.5: Wirkung gegen Epilachna varivestis:
Etwa 15 - 20 cm hohe Phaseolus vulgaris-Pflanzen (Buschbohnen) werden mit wässrigen, den zu prüfenden Wirkstoff in einer Konzentration von 800 ppm enthaltenden Emulsions-Zubereitungen besprüht. Nach dem Antrocknen des Sprühbelages werden pro Pflanze 5 Larven von Epilachna varivestis (Mexikanischer Bohnenkäfer) im 4. larvalen Stadium angesetzt. Ueber die infestierten Pflanzen wird ein Plastikzylinder gestülpt, der mit einem Kupfer-Gazedeckel abgedeckt ist. Der Versuch wird bei 28°C und 60 % relativer Luftfeuchtigkeit durchgeführt.

Nach 2 und 3 Tagen wird die Mortalität in Prozenten bestimmt. Zur Auswertung hinsichtlich allfälligem Frass-Schaden (Antifeeding-Effekt), Entwicklungs- und Häutungsstörungen werden die Versuchstiere während weiterer 3 Tage beobachtet.

Verbindungen der Formel I gemäss Beispiel 1 zeigen gute Wirkung in obigem Test.

Beispiel 3.6: Ovizide Wirkung auf Heliothis virescens:
Entsprechende Mengenanteile einer benetzbaren pulverförmigen Formulierung, enthaltend 25 Gew.% des zu prüfenden Wirkstoffes, werden mit jeweils so viel Wasser vermischt, dass sich eine wässrige Emulsion mit einer Wirkstoffkonzentration von 800 ppm ergibt.

In diese wirkstoffhaltigen Emulsionen werden eintägige Eigelege von Heliothis auf Cellophan während drei Minuten eingetaucht und dann auf Rundfilter abgenutscht. Die so behandelten Gelege werden in Petrischalen ausgelegt und in der Dunkelheit aufbewahrt. Nach 6 bis 8 Tagen wird die Schlupfrate im Vergleich zu unbehandelten Kontrollen festgestellt.

Verbindungen der Formel I gemäss Beispiel 1 zeigen gute Wirkung in obigem Test.

Beispiel 3.7: Wirkung auf Laspeyresia pomonella (Eier):
Abgelegte Eier von Laspeyresia pomonella, die nicht älter als 24 Stunden sind, werden auf Filterpapier für 1 Minute in eine acetonisch-wässrige Lösung, enthaltend 800 ppm des zu prüfenden Wirkstoffes, eingetaucht. Nach dem Antrocknen der Lösung werden die Eier in Petrischalen ausgelegt und bei einer Temperatur von 28°C belassen. Nach 6 Tagen wird der prozentuale Schlupf aus den behandelten Eiern bewertet und die Mortalität in Prozenten bestimmt.

Verbindungen der Formel I gemäss Beispiel 1 zeigen gute Wirkung in obigem Test.

Beispiel 3.8: Reproduktions-Beeinflussung von Anthonomus grandis:
Adulte Anthonomus grandis, die nach dem Schlupf nicht älter als 24 Stunden waren, werden in Gruppen zu jeweils 25 Käfern in Käfige mit Gitterwänden überführt. Die mit den Käfern besetzten Käfige werden sodann während 5 bis 10 Sekunden in eine acetonische Lösung, enthaltend 0,1 Gew.% des zu prüfenden Wirkstoffes, eingetaucht. Nachdem die Käfer wieder trocken sind, werden sie zur Kopulation und Eiablage in abgedeckte und Futter enthaltende Schalen eingesetzt. Abgelegte Eier werden zwei- bis dreimal wöchentlich mit fliessendem Wasser ausgeschwemmt, gezählt, durch zwei- bis dreistündiges Einlegen in ein wässriges Desinfektionsmittel desinfiziert und dann in Schalen, die eine geeignete Larvaldiät enthalten, deponiert. Nach 7 Tagen wird untersucht, ob sich aus den deponierten Eiern Larven entwickelt haben.

Zur Ermittlung der Dauer des die Reproduktion beeinflussenden Effektes der zu prüfenden Wirkstoffe wird die Eiablage der Käfer während eines Zeitraums von etwa 4 Wochen überprüft. Die Bonitierung erfolgt anhand der Verminderung der Anzahl abgelegter Eier und der daraus geschlüpften Larven im Vergleich zu unbehandelten Kontrollen.

Verbindungen der Formel I gemäss Beispiel 1 zeigen eine gute reproduktionsreduzierende Wirkung in obigem Test.

Beispiel 3.9: Wirkung gegen Anthonomus grandis (Adulte)
Zwei eingetopfte Baumwollpflanzen im 6-Blattstadium werden jeweils mit einer wässrigen benetzungsfähigen Emulsions-Zubereitung enthaltend 400 ppm des zu prüfenden Wirkstoffes besprüht. Nach dem Antrocknen des Spritzbelages (nach etwa 1,5 Stunden) wird jede Pflanze mit 10 adulten Käfern (Anthonomus grandis) besiedelt. Plastikzylinder, deren obere Oeffnungen mit Gaze abgedeckt sind, werden dann über die behandelten, mit den Testtieren besiedelten Pflanzen gestülpt, um ein Abwandern der Käfer zu verhindern. Die so behandelten Pflanzen werden bei 25°C und etwa 60 % relativer Luftfeuchtigkeit gehalten. Die Auswertung erfolgt nach 2, 3, 4 und

5 Tagen unter Zugrundelegung der prozentualen Mortalität der eingesetzten Testtiere (% Rückenlage) sowie der Antifeedant-Wirkung gegenüber unbehandelten Kontrollansätzen.

Verbindungen der Formel I gemäss Beispiel 1 zeigen gute Wirkung in obigem Test.

Beispiel 3.10: Insektizide Frassgift-Wirkung gegen Plutella xylostella

Eingetopfte Chinakohlpflanzen (Topfgrösse 10 cm Durchmesser) im 4-Blatt-Stadium werden mit wässrigen Wirkstoffemulsionen besprüht, die den Wirkstoff in einer Konzentration von 0,8 ppm enthalten und auf den Pflanzen antrocknen.

Nach zwei Tagen werden die behandelten Chinakohlpflanzen mit je 10 Plutella xylostella-Larven im zweiten larvalen Stadium besiedelt. Der Versuch wird bei 24°C und 60 % relativer Luftfeuchtigkeit im Dämmerlicht durchgeführt. Die Bonitur erfolgt nach 2 und 5 Tagen; es wird die %-Mortalität der Larven bestimmt.

Verbindungen der Formel I gemäss Beispiel 1 zeigen gute Wirkung in obigem Test.

Beispiel 3.11: Wirkung gegen Zecken - Benetzungstest/Eiablagehemmung

Als Testtiere werden mit Blut vollgesogene adulte Weibchen der Rinderzecke Boophilus microplus verwendet. Es werden je 10 Zecken eines OP-resistenten Stammes (z.B. vom Biarra-Stamm) und eines normal empfindlichen Stammes (z.B. vom Yeerongpilly-Stamm) behandelt. Die Zecken werden auf mit Doppelklebeband bezogenen Platten fixiert und mit einer Baumwoll-Watteschicht bedeckt, auf die 5 ml einer wässrigen Emulsion bzw. Lösung enthaltend 800 ppm der zu untersuchenden Verbindung aufgetropft werden. Nach einer Einwirkungszeit von 1 Stunde wird die Watteschicht entfernt und die Zecken ca. 12 Stunden trocknen gelassen. Anschliessend werden die Zecken in einem klimatisierten Raum bei konstanten Bedingungen aufbewahrt

(28°C und 90 % relative Luftfeuchtigkeit). Die Auswertung erfolgt nach drei Wochen. Es wird die %-Mortalität der Testtiere sowie die prozentuale Hemmung der Ablage von fertilen Eiern gegenüber unbehandelten Kontrollen ermittelt.

Verbindungen der Formel I gemäss Beispiel 1 zeigen gute Wirkung in obigem Test.

0221847

Patentansprüche

1. Salz eines Benzoylharnstoffes der Formel I

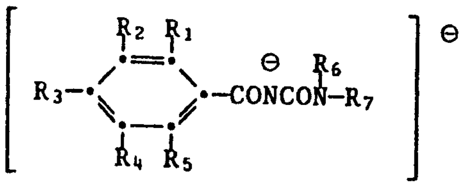 $X^{\oplus}$ (I),

worin

$R_1$ bis $R_5$ unabhängig voneinander Wasserstoff, Halogen oder $C_1$-$C_6$-Alkoxy,

$R_6$ Wasserstoff oder $C_1$-$C_6$-Alkyl,

$R_7$ gegebenenfalls substituiertes Phenyl, Naphthyl, Pyridyl, Pyridazyl, Pyrimidinyl oder Pyrazinyl und

$X^{\oplus}$ ein anorganisches oder organisches Kation bedeuten.

2. Eine Verbindung gemäss Anspruch 1, worin

$R_1$ Fluor, Chlor oder Methoxy,

$R_2$ bis $R_5$ unabhängig voneinander Wasserstoff, Fluor, Chlor oder Methoxy,

$R_6$ Wasserstoff oder Methyl,

$R_7$ gegebenenfalls substituiertes Phenyl und

$X^{\oplus}$ ein Kation der Alkalimetalle,

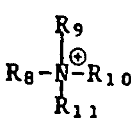 oder 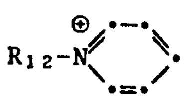 ,

$R_8$ bis $R_{11}$ unabhängig voneinander Wasserstoff $C_1$-$C_{20}$-Alkyl, Benzyl oder Phenyl und

$R_{12}$ Wasserstoff oder $C_1$-$C_{20}$-Alkyl bedeuten.

3. Verbindung gemäss Anspruch 2, worin

$R_1$ Fluor, Chlor oder Methoxy,

$R_2$ bis $R_5$ unabhängig voneinander Wasserstoff, Fluor, Chlor oder Methoxy,

$R_6$ Wasserstoff oder Methyl,

$R_7$ gegebenenfalls substituiertes Phenyl und

$X^{\oplus}$ ein Kation der Alkalimetalle oder $(CH_3)_4N^{\oplus}$, $(C_2H_5)_4N^{\oplus}$, $(n\text{-}C_3H_7)_4N^{\oplus}$, $(i\text{-}C_3H_7)_4N^{\oplus}$, $(n\text{-}C_4H_9)_4N^{\oplus}$,

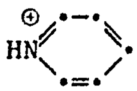 , $CH_3\text{-}(CH_2)_{8\text{-}15}\text{-}$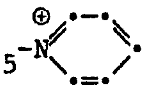 oder

$CH_3\text{-}(CH_2)_{8\text{-}15}\text{-}N^{\oplus}(CH_3)_3$ bedeuten.

4. Eine Verbindung gemäss Anspruch 3, worin

$R_1$ Fluor, Chlor oder Methoxy,

$R_2$ bis $R_5$ unabhängig voneinander Wasserstoff, Fluor, Chlor oder Methoxy,

$R_6$ Wasserstoff,

$R_7$ ein Phenyl substituiert durch ein oder mehrere Halogenatome, Halogenalkyl-, Halogenalkoxy- oder Dialkylaminogruppen, durch einen durch ein oder mehrere Nitro, Halogen, Halogenalkyl oder Halogenalkoxy substituierten Phenoxy- oder Pyridyloxyrest oder durch die Gruppen

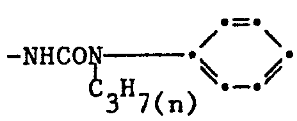 , $3,4\text{-}O\text{-}CF_2\text{-}CF_2\text{-}O\text{-}$ oder $3,4\text{-}O\text{-}CF_2\text{-}CFCl\text{-}O\text{-}$

und

$X^{\oplus}$ $Na^{\oplus}$, $K^{\oplus}$, oder $(CH_3)_4N^{\oplus}$, $(C_2H_5)_4N^{\oplus}$, $CH_3\text{-}(CH_2)_{15}\text{-}N^{\oplus}(CH_3)_3$ oder $(n\text{-}C_4H_9)_4N^{\oplus}$ bedeuten.

5. Verbindung gemäss Anspruch 4 der Formel

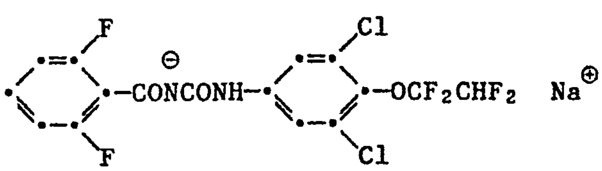 .

6. Verbindung gemäss Anspruch 4 der Formel

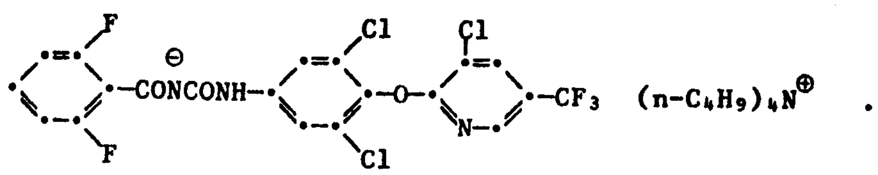

7. Verbindung gemäss Anspruch 4 der Formel

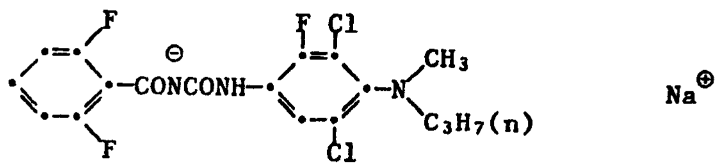

8. Verbindung gemäss Anspruch 4 der Formel

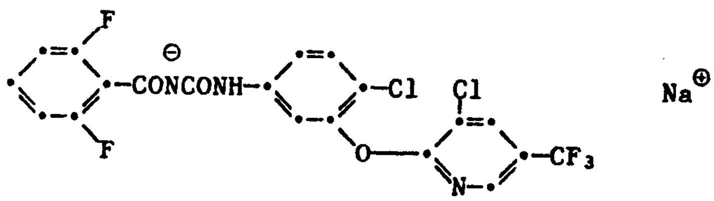

9. Verbindung gemäss Anspruch 4 der Formel

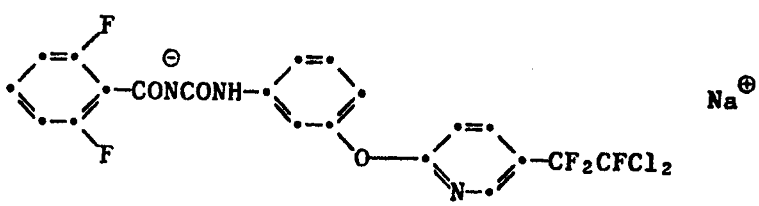

10. Verbindung gemäss Anspruch 4 der Formel

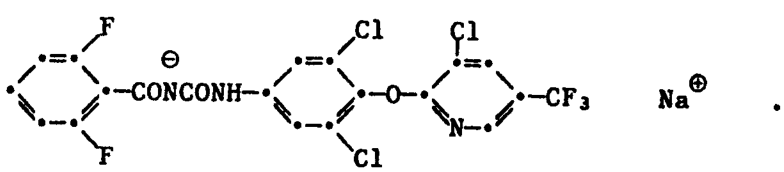

11. Verfahren zur Herstellung einer Verbindung gemäss einem der Ansprüche 1 bis 10, dadurch gekennzeichnet, dass man eine Verbindung der Formel II

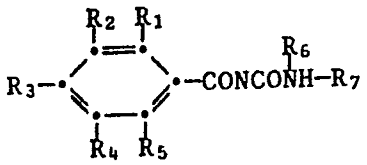 (II),

worin $R_1$-$R_7$ die in Anspruch 1 angegebene Bedeutung haben, mit einem Metallalkanolat oder einem Ammoniumhydroxid der Formel III $X^{\oplus}OH^{\ominus}$ (III), worin $X^{\oplus}$ ein organisches Kation bedeutet, umsetzt.

12. Schädlingsbekämpfungsmittel, welches als aktive Komponente eine Verbindung gemäss einem der Ansprüche 1 bis 10 zusammen mit geeigneten Trägern und/oder anderen Zuschlagstoffen enthält.

13. Verwendung einer Verbindung gemäss einem der Ansprüche 1 bis 10 zur Bekämpfung von Insekten und Vertretern der Ordnung Akarina.

14. Verwendung gemäss Anspruch 13 zur Bekämpfung larvaler Stadien pflanzenschädigender Insekten.

15. Verwendung gemäss Anspruch 13 zur Bekämpfung von Zecken.

16. Verwendung gemäss Anspruch 15 der Verbindungen gemäss einem der Ansprüche 6 bis 9.

17. Verfahren zur Bekämpfung von Insekten und Vertretern der Ordnung Akarina, dadurch gekennzeichnet, dass man die Schädlinge bzw. deren verschiedene Entwicklungsstadien oder deren Aufenthaltsort mit einer pestizid wirksamen Menge einer Verbindung der Formel I gemäss einem der Ansprüche 1 bis 10 oder mit einem Mittel enthaltend neben Zusatz- und Trägerstoffen eine pestizid wirksame Menge dieser Verbindung, in Kontakt bringt oder behandelt.

FO 7.5 EIC/cs*/eg*